# EUROPEAN PATENT APPLICATION

(11) **EP 4 220 177 A2**
(43) Date of publication of application: **02.08.2023**
(21) Application number: 23153835.6
(22) Date of filing: 30.01.2023
(51) Int. Cl.: G01N 33/86

(54) **IDENTIFYING DIRECT ORAL FACTOR XA INHIBITORS**

(30) Priority: 31.01.2022 US 202263304923 P; 16.06.2022 US 202263352829 P; 18.01.2023 US 202318098422
(71) Applicant: Instrumentation Laboratory Company, Bedford, MA 01730 (US)
(72) Inventor: Schwaiger, Martin, San Diego (US); Kholmukhamedov, Andaleb, San Diego (US); Bottenus, Ralph, San Diego (US)
(74) Representative: Müller-Boré & Partner Patentanwälte PartG mbB

(57) **Abstract**

An example method includes the following operations: obtaining a clotting parameter based on a mixture including a test sample and a reagent, where the reagent includes a polycation; using the clotting parameter to identify at least one of a potential presence of a direct oral factor Xa inhibitor in the test sample or an estimated concentration of the direct oral factor Xa inhibitor in the test sample; and reporting a result that is based on the potential presence or the estimated concentration.

## Description

This specification relates generally to example processes for identifying a potential presence of, and for estimating an amount of, a direct oral factor Xa inhibitor in a test sample, and to example reagents, kits, cartridges, and devices for use in those processes.

Direct factor Xa inhibitors include anticoagulant drugs used to prevent blood clots. Direct factor Xa inhibitors may be administered orally at the time of a medical event, such as a stroke, or on a regular basis as a thromboprophylaxis regimen.

Taking direct factor Xa inhibitors can increase a patient's risk of bleeding, particularly after trauma, during surgery, and during invasive interventions. In order to treat the patient in a point of care (POC) setting, it may be necessary to determine whether the patient is using a direct factor Xa inhibitor.

An example method includes the following operations: obtaining a clotting parameter based on a mixture including a test sample and a reagent, where the reagent includes a polycation; using the clotting parameter to identify at least one of a potential presence of a direct oral factor Xa inhibitor in the test sample or an estimated concentration of the direct oral factor Xa inhibitor in the test sample; and reporting a result that is based on the potential presence or the estimated concentration. The example method may include one or more of the following features, either alone or in combination.

The direct oral factor Xa inhibitor may include apixaban, rivaroxaban, betrixaban, or edoxaban. The result may include an identity of the direct oral factor Xa inhibitor. The result may include the estimated concentration of the direct oral factor Xa inhibitor. The method may include obtaining the clotting parameter based on a mixture of the test sample and the reagent. The reagent may also include a diluted tissue factor. The polycation may be at an assay concentration within a range of 1 to 100 micrograms/milliliter (µg/mL). The polycation may include at least one of polybrene, protamine, polylysine, polyamine, spermine, putrescin, cadaverin, DEAE (diethylaminoethyl) dextran, or DEAE cellulose. The clotting parameter may be at least one of clot formation time or time to clot.

An example system includes a cartridge configured to receive a test sample. The cartridge may contain a reagent to mix with the test sample in the cartridge to form a mixture of the test sample and the reagent. The reagent may include a diluted tissue factor and a heparin inhibitor that includes a polycation. One or more processing devices may be configured to perform the following operations: using a clotting parameter based on the mixture of the test sample and the reagent to identify at least one of a potential presence of a direct oral factor Xa inhibitor in the test sample or an estimated concentration of the direct oral factor Xa inhibitor in the test sample; and reporting a result that is based on the potential presence or the estimated concentration. The example system may include one or more of the following features, either alone or in combination.

The direct oral factor Xa inhibitor may include apixaban, rivaroxaban, betrixaban, or edoxaban. The result may include an identity of the direct oral factor Xa inhibitor. The result may include the estimated concentration of the direct oral factor Xa inhibitor. The polycation may include at least one of polybrene, protamine, polylysine, polyamine, spermine, putrescin, cadaverin, DEAE (diethylaminoethyl) dextran, or DEAE cellulose. The polycation may be at an assay concentration within a range of 1 to 100 micrograms/milliliter (µg/mL). The reagent may include calcium chloride.

The clotting parameter may include at least one of a time to clot for the test sample or a clot formation time for the test sample. Or, the clotting parameter may include the time to clot for the test sample and the clot formation time for the test sample. Or, the clotting parameter may include a clotting time for the test sample and the clot formation time for the test sample.

An example reagent includes a diluted tissue factor, a heparin inhibitor including a polycation, and calcium chloride. The example reagent may include one or more of the following features, either alone or in combination.

The polycation may include at least one of polybrene, protamine, polylysine, polyamine, spermine, putrescin, cadaverin, DEAE (diethylaminoethyl) dextran, or DEAE cellulose. The reagent may be usable in a clotting assay, The polycation may be at an assay concentration within a range comprising 1 to 100 micrograms/milliliter (µg/mL).

An example kit includes a reagent including a diluted tissue factor, a heparin inhibitor that includes a polycation, and calcium chloride, The kit is for performing a clotting assay manually based on a mixture of a test sample and the reagent.

Any two or more of the features described in this specification, including in this summary section, can be combined to form implementations not specifically described herein.

The systems and processes described herein, or portions thereof, may be implemented as one or more apparatus or as a method and may include one or more processing devices and computer memory to store executable instructions to implement processes and algorithms. The systems, processes, and substances, including but not limited to apparatus, methods, and/or reagents, described herein may be configured, for example, through design, manufacture, construction, composition, arrangement, placement, programming, operation, activation, deactivation, and/or control.

The details of one or more implementations are set forth in the accompanying drawings and the description below. Other features, objects, and advantages will be apparent from the description and drawings, and from the claims.

The figures show:
Fig. 1 is a flowchart showing an example process for identifying a potential presence, or estimating an amount of, direct oral factor Xa inhibitors in a test sample.
Fig. 2 is a graph showing an example relationship between apixaban concentration and the time it takes for a mixture of test sample and reagent to clot.
Fig. 3 is a graph showing an example relationship between rivaroxaban concentration and the time it takes a mixture of test sample and reagent to clot.
Fig. 4 shows an example of the apixaban dose-response-curve.
Fig. 5 shows an example of the rivaroxaban dose-response-curve.
Fig. 6 shows an example graph of the time it takes a mixture of test sample and reagent also containing heparin to clot.

Like reference numerals in different figures indicate like elements.

Described herein are example systems, devices, materials, and processes for use with a clotting assay to identify a potential presence of, and in some cases to estimate an amount of, direct oral factor Xa inhibitors in a test sample. Direct oral factor Xa inhibitors include anti-Xa DOACs (direct oral anticoagulants), examples of which include, but are not limited to, apixaban, rivaroxaban, betrixaban, and edoxaban. Direct oral factor Xa inhibitors include other drugs of the same substance class as these.

The clotting assay uses a reagent to initiate the clotting of a test sample, such as whole blood or a sample derived from whole blood (e.g., plasma). A low tissue factor assay is an example of a clotting assay. A low tissue factor assay uses a reagent that has a reduced tissue factor concentration - in comparison to that used in a standard prothrombin time (PT) assay - for reduced activation of the extrinsic pathway. This increases the sensitivity of the low tissue factor assay to anticoagulant effects. The low tissue factor assay monitors the clotting of a mixture of the test sample and the reagent and determines clotting parameters based on that clotting.

Examples of clotting parameter(s) that are determined by the low tissue factor assay are based on parameters relating to clotting of the mixture of the sample. Examples of these parameters include time-to-clot (TTC) parameters. Generally, the TTC parameters are sensitive to anti-Xa DOACs. Graphs for assay concentrations of direct oral factor Xa inhibitors versus TTC are generally linear.

The TTC parameters can be different on different clinical analyzer systems and for different low tissue factor assays. For example, TTC parameter(s) for low tissue factor assays performed using the ROTEM^{®} *sigma* and the ROTEM^{®} *delta* systems by Werfen^{®} include clotting time (CT) (where CT is the latency time from adding reagent to sample until the clot starts to form); TTC parameter(s) for low tissue factor assays performed using the TEG^{®} 6s system by Haemonetics^{®} include reaction time (R) and clotting time (CT); and TTC parameter(s) for low tissue factor assays performed using the Quantra^{®} system by the Hemosonics^{®} system include clot time (CT).

In addition to the parameters described above, other clotting parameters that may be determined by the low tissue factor assay include, but are not limited to, clot formation time (CFT), where CFT is the time from clotting time until a clot firmness of 20 millimeters has been reached, and clot amplitude parameters, such as A5, A10, A15, and A20, which describe clot firmness (or amplitude) obtained after 5, 10, 15 or 20 minutes, respectively, beginning from CFT. Additional clotting parameters not listed here may also be determined using the low tissue factor assay.

An example of the reagent that may be used in the low tissue factor assay (referred to below as "the assay") to initiate clotting of the test sample includes the following constituents: a diluted tissue factor ("the tissue factor"), calcium chloride (CaCl₂), and a heparin inhibitor. The tissue factor activates the extrinsic pathway; the heparin inhibitor reduces or eliminates the sensitivity of the assay to heparin or at least certain concentrations of heparin in the test sample; and CaCl₂ recalcifies the sample as required for blood clotting to occur. An example of a type of heparin inhibitor that may be used in the reagent is polycation. An example of a polycation that may be used in the reagent includes, but is not limited to, polybrene. Polybrene can cause the reagent to have increased sensitivity to apixaban relative to reagents that do not include polybrene. In this regard, apixaban has heretofore been a challenging direct oral factor Xa inhibitor to identify using clotting assays, particularly at low concentrations (for example, at concentrations of 25 nanograms/milliliter {ng/mL} or less). The increased sensitivity of the reagent to apixaban enables low tissue factor assay to address the challenge of identifying apixaban at low concentrations.

Examples of other polycations that may be included in the reagent are water-soluble and may include, but are not limited to, one or more of the following: protamine, polylysine, polyamine(s), spermine, putrescin, cadaverine, and DEAE (diethylaminoethyl) dextran. An example of a polycation that may be used in the reagent and that is not water-soluble is DEAE cellulose.

In some implementations, the reagent used in the assay may include the tissue factor at an assay concentration within a range of 1 to 1000 picomolar (picomoles/liter). The reagent used in the assay may include the CaCl₂ at an assay concentration within a range of 9 to 15 millimolar (e.g., 12 millimolar), 5 to 30 millimolar, or 5 to 50 millimolar. In some implementations, the assay may include the polybrene, or more generally one or more polycations, at an assay concentration within a range of 1 to 100 micrograms/milliliter (µg/mL) or 1 to 50 µg/mL In some implementations, the reagent used in the assay may include the polybrene or, more generally, one or more polycations, at assay concentration/s that inhibit up to 3 U/mL (units-per-milliliter) of unfractionated heparin or heparinoids. One or more other polycations, such as those described above, may be present in the reagent at similar or identical assay concentrations to those described herein for polybrene.

The reagent may be in solid form or liquid form. Solid forms, such as beads, pellets, lyophilized forms, dried layers, lyocake, or a surface coating, to name a few examples, may have advantages over liquid form in terms of stability over time. In some cases, liquids are ready to use and therefore may be preferred in cases where manual systems are used. In use, the reagent may be included, e.g., in a prepackaged kit or in a cartridge used to perform the assay.

Examples of direct oral factor Xa inhibitors that may be identified, and the assay concentrations of which may be estimated, using the processes described herein include those described above. However, the processes described herein can be to detect and estimate any direct oral factor Xa inhibitors and are not limited to use with the direct oral factor Xa inhibitors listed here or described herein.

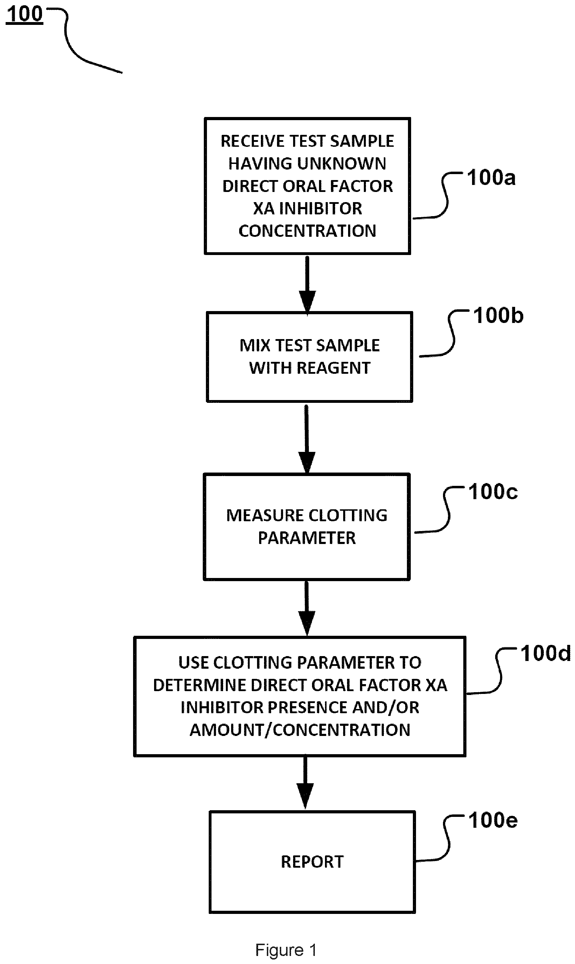

Fig. 1 shows an example process 100 that uses the low tissue factor assay for identifying the potential presence of a direct oral factor Xa inhibitor such as an anti-Xa DOAC, and in some cases for estimating an amount of, the direct oral factor Xa inhibitor in the sample. The example low tissue factor assay uses the reagent described herein that includes a diluted tissue factor, a heparin inhibitor that is typically a polycation, and CaCl₂. Process 100 may be performed using a diagnostic testing / clinical analyzer system or a kit such as those described herein. However, process 100 is not limited to use with any particular automated or manual system or testing configuration.

In this example, process 100 includes receiving (100a) a test sample having an unknown anti-Xa DOAC at an unknown concentration. The test sample may be received in a diagnostic testing / clinical analyzer system or a manual testing system. The test sample may be any of the types of test samples described herein. The system performs a low tissue factor assay on the test sample. This includes causing the test sample to contact a reagent such as the example reagents described herein.

The test sample mixes (100b) with the reagent. The mixing may be performed automatically or manually by physically moving the test sample and the reagent using a mixing element mechanism, e.g., agitation, flow, or the like. The resulting liquid, which is a mixture of the sample and the reagent, begins to clot. The clotting parameters(s), such as TTC, CFT, and/or amplitudes, of clots formed from the liquid may be measured (100c) by the diagnostic testing / clinical analyzer system or manually.

The clotting parameter(s) may be used (100) to determine the presence, of and to estimate an assay concentration of, the anti-Xa DOACs in the test sample. In some implementations, TTC alone may be used. In some implementations, CFT may be used alone. In some implementations, a combination of TTC (e.g., CT) and CFT, or TTC and amplitude parameters may be used. Other combinations of clotting parameters not specifically mentioned here may also be used.

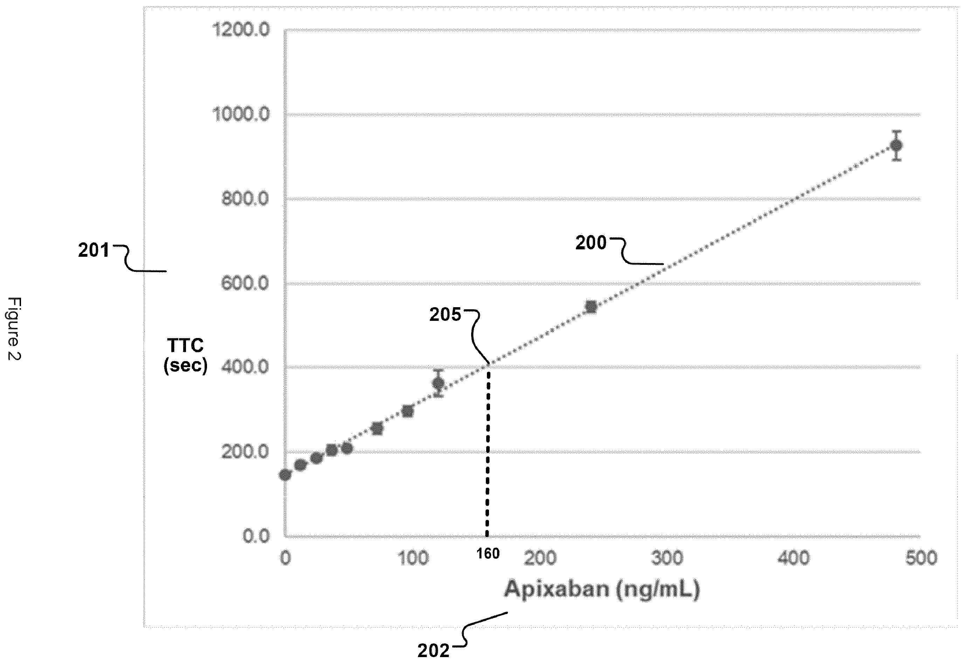

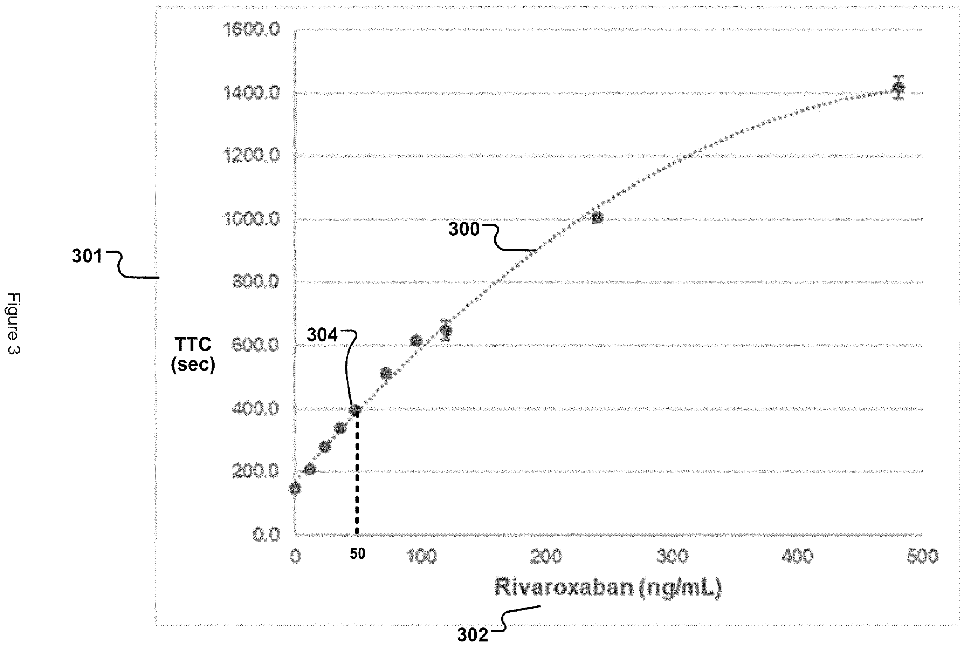

A diagnostic testing / clinical analyzer system may store, or have access to remote storage, that includes data or algorithms relating to, or generated based on, clotting parameters for ranges of assay concentrations of anti-Xa DOACs. The data may be stored in tables or other structures such as look-up tables (LUTs), arrays, linked lists, or the like. Examples of the data include, but are not limited to, the plots of Figs. 2 and 3 described below.

Fig. 2 is an example plot 200 that shows that the relationship between TTC 201 in seconds ("sec") and an apixaban concentration 202 in a sample is linear, at least over the range shown, which is between 0 and 500 ng/mL, with the last apixaban concentration in this example being at 481 ng/mL. Fig. 3 is an example plot 300 that shows that the relationship between TTC 301 and rivaroxaban concentration 302 in a test sample is linear over at least over the range shown, which is between 0 and 500 ng/mL, with the last rivaroxaban concentration in this example being at 498 ng/mL.

The clotting parameter(s) are used to determine (100d) the presence and/or amount of anti-Xa DOAC in the sample. The amount may be quantified, for example, if the DOAC present in the sample is known beforehand. In an example, the TTC is compared to the stored data (e.g., Figs. 2 and 3) for one or more anti-Xa DOACs in order to identify the potential presence of an anti-Xa DOAC in the sample and/or to estimate a potential concentration of the anti-Xa DOAC in the sample.

Since the TTC for a sample may match data for more than one anti-Xa DOAC, process 100 may not identify only one type of anti-Xa DOAC, but rather may identify multiple possible anti-Xa DOACs in a test sample and identify the estimated concentration(s) for some types of anti-Xa DOACs such as, but not limited to, apixaban and edoxaban. For example, referring to Fig. 2, if the measured clotting time is 400 seconds (205), then potentially, the test sample includes apixaban at a concentration of about 160 ng/mL. For example, referring to Fig. 3, if the measured clotting time is 400 seconds (304), then potentially, the test sample includes rivaroxaban at a concentration of about 50 ng/mL.

If, however, the test sample is known to contain a particular type of anti-Xa DOAC (information that may be entered into the diagnostic testing / clinical analyzer system beforehand), then process 100 may estimate a concentration of only that particular type of anti-Xa DOAC in the test sample. For example, process 100 may only compare the measured TTC to data for that type of anti-Xa DOAC.

Instead of, or in addition to, using tables like those of Figs. 2 and 3, the clotting parameter(s) may be input to an algorithm, which provides an output indicating the presence of an anti-Xa DOAC in the sample and, in some cases, an estimate of the amount of anti-Xa DOAC in the test sample. In some examples, the algorithms can estimate the quantity of DOAC presence based on calibration data. Calibration data may be determined beforehand by performing clotting assays on test samples having known, and different, concentrations of, anti-Xa DOACs that do not include heparin or by training a model using predefined data.

Examples of the types of algorithms that may be used include machine learning algorithms. Examples of the types of algorithms that may be used are described in U.S. Patent Application No. 17/480,485 ("Detecting Oral Anticoagulants Or Intravenous Direct Thrombin Inhibitors in a Blood Sample") that was filed on September 21, 2021, the contents of which are incorporated herein by reference.

Process 100 reports (100e) of the results - e.g., the identity of a DOAC and/or a concentration of the DOAC. This report may be, for example, graphical or alphanumeric. The report may be on a user interface of the diagnostic testing / clinical analyzer system. For example, the report may state or indicate that the test sample may contain apixaban at a concentration of about 160 ng/mL or that the test sample may contain rivaroxaban at a concentration of about 50 ng/mL. For reasons noted above, the report may not definitively indicate which anti-Xa DOAC is present in the test sample, but rather may indicate which anti-Xa DOACs potentially are present with or without their estimated concentrations. If the DOAC is unknown, the report may contain an estimate of its concentration, which may not be semi-quantitative. However, if the DOAC is known (e.g. rivaroxaban, apixaban, or the like) then the report may include a relatively accurate estimate (e.g., semi-quantitative) of the DOAC concentration.

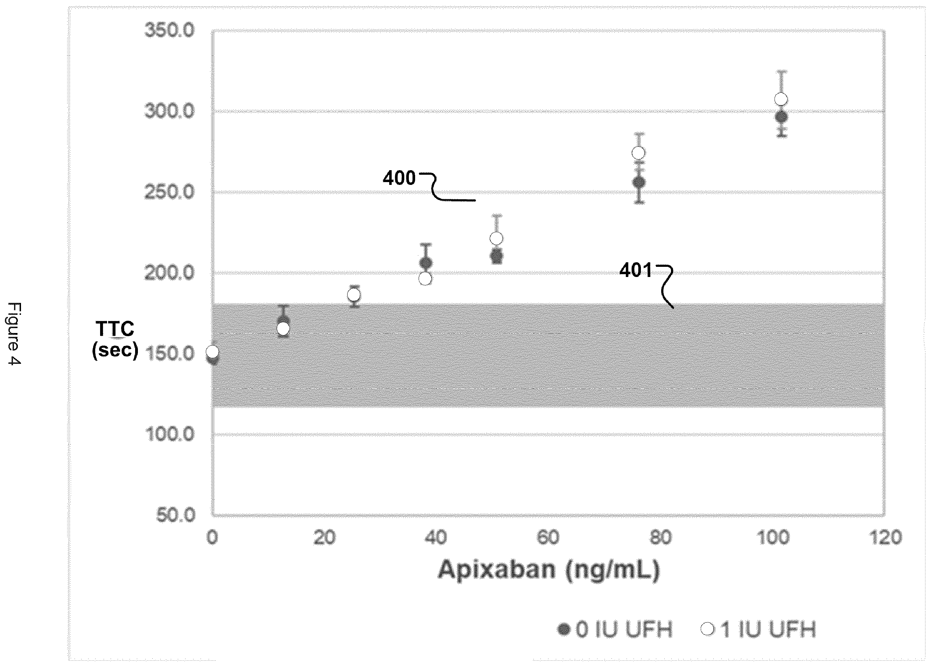

The plots of Figs. 4 to 6, which are described below, are associated with various experiments using the low tissue factor assay and the example reagents described herein containing the tissue factor, CaCl₂, and a heparin inhibitor, such as polybrene or other polycation.

The experiment associated with Fig. 4 is used to show that, with a typical heparin concentration that may be present in a test sample, the assay described herein is not influenced by the presence of heparin. In this regard, Fig. 4 shows points in region 400 of a plot like that of Fig. 2 in a case where a test sample includes apixaban and one international unit (IU) of unfractionated heparin (that is "1 IU UFH") and in a case where the test sample includes apixaban and no heparin (that is "0 IU UFH"). As shown, the heparin inhibitor (e.g., polybrene or other polycation) in the reagent ensures that the plot remains substantially linear in region 400. Also shown is region 401, referred to as a normal range, in which the low tissue factor assay produces results for a test sample that are independent of whether the test sample contains apixaban. So, in some implementations, identifying apixaban may not be implemented within region 401. In this experiment, region 401 was determined using test samples from 30 donors not taking apixaban.

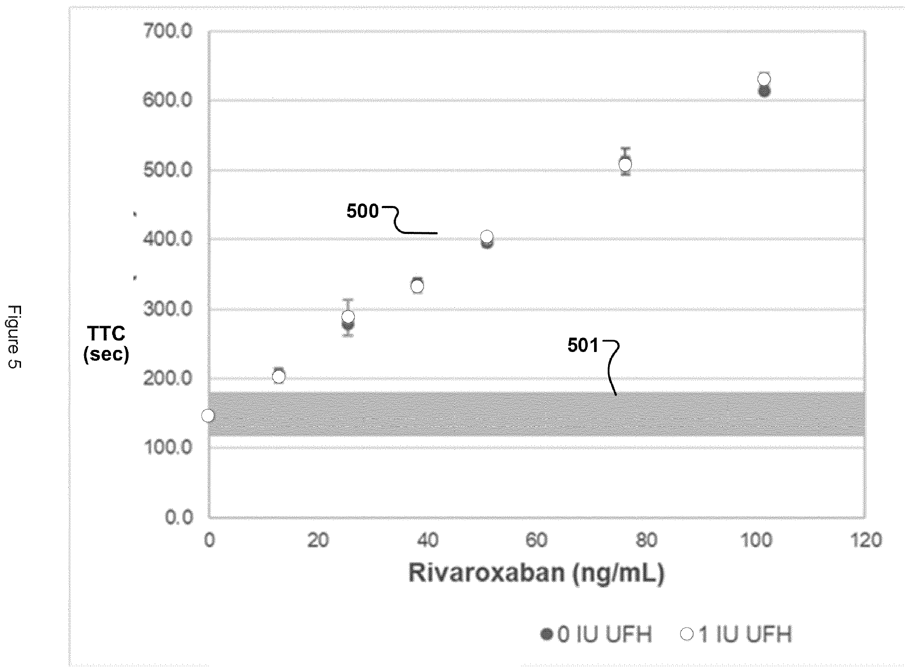

The experiment associated with Fig. 5 is the same as that associated with Fig. 4 except for rivaroxaban instead of apixaban. As shown, the heparin inhibitor (e.g., polybrene or other polycation) in the reagent ensures that the plot remains substantially linear in region 500. Region 501 is the normal range described above.

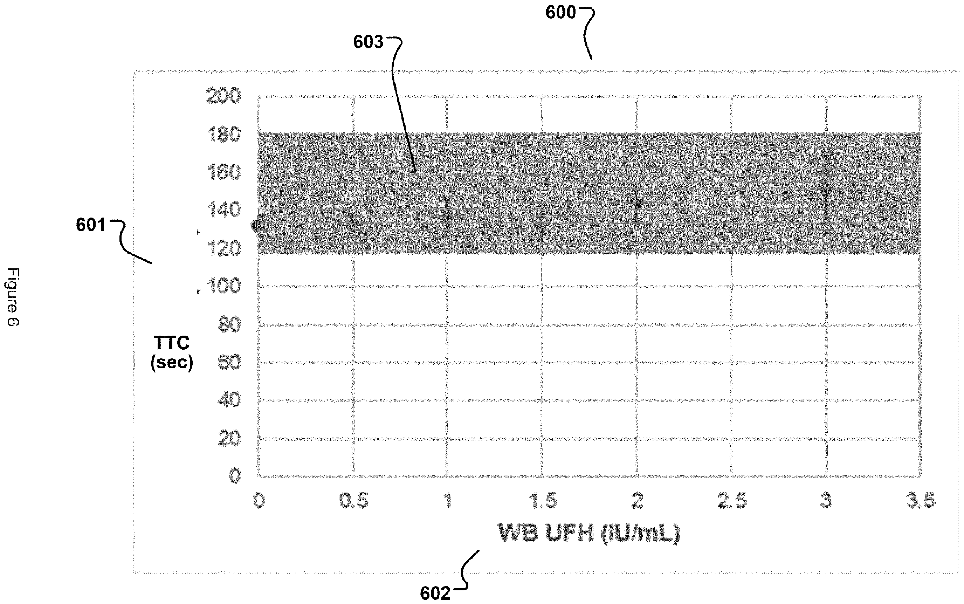

The experiment associated with Fig. 6 is used to show that, with polybrene added to the reagent, the assay becomes insensitive to heparin up to a heparin assay concentration of 3 U/ml. In this regard, Fig. 6 shows an example plot 600 of TTC 601 versus a heparin assay concentration 602 (whole blood unfractionated heparin - WB UFH) for samples that contain at least one anti-XA DOAC and that contain unfractionated heparin having assay concentrations of 0 to 3 lU/mL Area 603 represents a reference range determined using 30 test samples that do not include heparin. The data points (similar to those of Figs. 4 and 5) remain in the reference range at least up to an assay concentration of 3 IU/mL heparin, which indicates that heparin does not interfere with the low tissue factor assay using the reagent described herein at least up to an assay concentration of 3 IU/mL heparin.

As noted, the example low tissue factor assays described herein may be implemented manually or as an automated assay using a panel of assays, which may be part of a manual or automated testing kit. For example, the kit may contain a cuvette, cartridge, tube, or another device in which the assay is to be performed, the reagent as described herein, and/or instructions for use.

As noted, the low tissue factor assay may be partially or fully automated. For example, an automated low tissue factor assay may be performed using tactile techniques ((e.g., rotational detection) as in the ROTEM^{®} *sigma* or the ROTEM^{®} *delta* systems and the TEG^{®} 6s system or non-tactile techniques, such as ultrasound detection in the Quantra^{®} system, optical detection, or a combination thereof.

An example system that may be used to implement the low tissue factor assay includes, but is not limited to, the system described in U.S. Patent No. 10,175,225 ("Blood Testing System and Method") that issued on January 8, 2019, the contents of which are incorporated herein by reference. U.S. Patent No. 10,175,225 describes a blood testing system that includes an analyzer console and one or more cartridges for performing tests, including a low tissue factor assay that may be used for identifying, and estimating an amount of, direct oral factor Xa inhibitors in a test sample using a reagent that includes a diluted tissue factor, CaCl₂, and a heparin inhibitor including a polycation, such as polybrene or other polycations described above.

An example cartridge used in the blood testing system may include one or more chambers for receiving/containing the test sample, the reagent(s), and/or the mixture of the test sample and the reagent(s). A clotting assay is performed on the mixture of the test sample and reagent to determine clotting characteristic(s) based on the mixture, such as TTC, CFT, and clot amplitude. The cartridge includes multiple fluid channels to allow for and facilitate fluidic movement of the sample or other materials in the cartridge, e.g., controlled by pressure or other mechanisms.

In another non-limiting example, the low tissue factor assay may be performed using the system described in U.S. Patent Publication No. 2018/0306774 ("Disposable System for Analysis of Hemostatic Function") that published on October 25, 2018, the contents of which are incorporated herein by reference. This patent publication describes the Quantra^{®} hemostasis analyzer.

The low tissue factor assays may have a turnaround time of 15 to 20 minutes in some examples, making them suitable for point-of-care (POC) applications.

The diagnostic test instruments / clinical analyzers and assays described herein may be controlled using computing systems or any other appropriate computing device having one or more processing devices, such as one or more microprocessors, one or more microcontrollers, or programmable logic such as one or more field-programmable gate arrays (FGPAs) or one or more application-specific integrated circuits (ASICs). The diagnostic test instruments / clinical analyzers described herein may execute one or more computer program products, e.g., one or more computer programs tangibly embodied in one or more information carriers, such as one or more non-transitory machine-readable media, to control the coagulation assays and to implement all or part of process 100 shown in Fig. 1.

Elements of different implementations described herein may be combined to form other implementations not specifically set forth above. Elements may be left out of the structures described herein without adversely affecting their operation. Furthermore, various separate elements may be combined into one or more individual elements to perform the functions described herein.

## Claims

1. A method comprising:
obtaining a clotting parameter based on a mixture comprising a test sample and a reagent, the reagent comprising a polycation;
using the clotting parameter to identify at least one of a potential presence of a direct oral factor Xa inhibitor in the test sample or an estimated concentration of the direct oral factor Xa inhibitor in the test sample; and
reporting a result that is based on the potential presence or the estimated concentration.

2. The method of claim 1, further comprising:
obtaining the clotting parameter based on a mixture comprising the test sample and the reagent, the reagent further comprising a diluted tissue factor.

3. The method of claim 1, wherein the result comprises the estimated concentration of the direct oral factor Xa inhibitor, and wherein optionally the polycation comprises at least one of polybrene, protamine, polylysine, polyamine, spermine, putrescin, cadaverin, DEAE (diethylaminoethyl) dextran, or DEAE cellulose.

4. The method of claim 1, wherein the clotting parameter comprises at least one of clot formation time or time to clot.

5. A system comprising:
a cartridge configured to receive a test sample, the cartridge containing a reagent to mix with the test sample in the cartridge to form a mixture comprised of the test sample and the reagent, the reagent comprising a diluted tissue factor and a heparin inhibitor comprising a polycation; and
one or more processing devices for performing operations comprising:
using a clotting parameter based on the mixture of the test sample and the reagent to identify at least one of a potential presence of a direct oral factor Xa inhibitor in the test sample or an estimated concentration of the direct oral factor Xa inhibitor in the test sample; and
reporting a result that is based on the potential presence or the estimated concentration.

6. The method of claim 1, or the system of claim 5, wherein the direct oral factor Xa inhibitor comprises apixaban, rivaroxaban, betrixaban, or edoxaban.

7. The method of claim 1, or the system of claim 5, wherein the result comprises
(i) an identity of the direct oral factor Xa inhibitor, or
(ii) the estimated concentration of the direct oral factor Xa inhibitor.

8. The system of claim 5, wherein the polycation comprises at least one of polybrene, protamine, polylysine, polyamine, spermine, putrescin, cadaverin, DEAE (diethylaminoethyl) dextran, or DEAE cellulose.

9. The method of claim 2, or the system of claim 5, wherein the polycation is at an assay concentration within a range of 1 to 100 micrograms/milliliter (µg/mL).

10. The system of claim 5, wherein the reagent comprises calcium chloride.

11. The system of claim 5, wherein the clotting parameter comprises at least one of a time to clot for the test sample or a clot formation time for the test sample; or wherein the clotting parameter comprises the time to clot for the test sample and the clot formation time for the test sample; or
wherein the clotting parameter comprises a clotting time for the test sample and the clot formation time for the test sample.

12. A reagent comprising:
a diluted tissue factor;
a heparin inhibitor comprising a polycation; and
calcium chloride.

13. The reagent of claim 12, wherein the polycation comprises at least one of polybrene, protamine, polylysine, polyamine, spermine, putrescin, cadaverin, DEAE (diethylaminoethyl) dextran, or DEAE cellulose.

14. The reagent of claim 12, wherein the reagent is usable in a clotting assay; and wherein the polycation is at an assay concentration within a range comprising 1 to 100 micrograms/milliliter (µg/mL).

15. A kit comprising:
the reagent of claim 12, the kit for performing a clotting assay manually based on a mixture of a test sample and the reagent.
